# EUROPEAN PATENT APPLICATION

(11) **EP 4 206 215 A1**
(43) Date of publication of application: **05.07.2023**
(21) Application number: 21846257.0
(22) Date of filing: 12.07.2021
(51) Int. Cl.: C07K 14/05, A61P 31/20, A61K 39/12

(54) **EBV-TARGETED ALLOGENEIC B CELL VACCINE AND PREPARATION METHOD THEREFOR**

(30) Priority: 24.07.2020 CN 202010728423
(71) Applicant: West China Hospital of Sichuan University, Chengdu, Sichuan 610041 (CN)
(72) Inventor: YANG, Hanshuo, Chengdu, Sichuan 610041 (CN); WEI, Yuquan, Chengdu, Sichuan 610041 (CN)
(74) Representative: Dolphin, Kirsty Mairi
(86) International application number: PCT/CN2021/105809
(87) International publication number: WO 2022/017217

(57) **Abstract**

The invention belongs to the field of biotechnology, and relates to an allogeneic B cell vaccine against various human-susceptible viruses and a preparation method therefor. The vaccine has anti-tumor and/or anti-viral preventive and/or therapeutic effects. Specifically, the present invention provides a B cell composition, wherein comprising an allogeneic B cell and a virus antigen, the B cell composition is irradiated with a certain dose of ionizing irradiation. The present invention also provides a B cell vaccine, comprising the above B cell composition. The invention also provides a preparation method for the B cell vaccine and a method and system for improving the antigen presentation ability of the B cell.

## Description

### Priority Application

. The present application claims priority from Chinese invention patent application CN2020107284230 "AN ALLOGENEIC B CELL VACCINE AND PREPARATION METHOD THEREFOR" filed on July 24, 2020, which is incorporated by reference in its entirety.

### Technical Field

. The present invention belongs to the field of biotechnology, and particularly relates to an allogeneic B cell vaccine against EB virus and/or EB virus antigen and a preparation method therefor.

### Background

. Epstein-Barr virus (EBV) is widely disseminated in the Chinese population. According to serological surveys, the positive rate of Epstein-Barr virus VCA-IgG antibody in Chinese children aged 3 to 5 is over 90%. Most young children have no obvious or only mild symptoms after infection. EBV is closely associated with a variety of malignancies, including nasopharyngeal carcinoma, which is highly prevalent in China, various lymphomas (Hodgkin's lymphoma (HD), non-Hodgkin's lymphoma (NHD), Burkitt's lymphoma (lymphoma in African Children), diffuse large B-cell lymphoma and post-transplant lymphoma, etc.), lethal midline granuloma (midline malignant reticulosis), infectious mononucleosis, and a small number of gastric cancers. It is estimated that there are hundreds of thousands of new EBV-positive cancer patients in China.

. Therapeutic tumor vaccine is a popular immunotherapy strategy, which achieves the purpose of treating tumors by activating the immune response in patients. Dendritic Cell (DC)-based therapeutic tumor vaccines are currently the most studied tumor vaccines. Past basic R&D and clinical practice have proven that vaccines prepared using antigen-presenting cells loaded with tumor antigens can produce objective clinical therapeutic effects. The world's first anti-tumor cell-based vaccine, Provenge, was approved by the US FDA in 2010 for the treatment of prostate cancer.

. Studies in recent years have shown that DC vaccines have good therapeutic effects on a variety of solid tumors, but DC cells are difficult to culture and expand in vitro, requiring individualized preparation, which has complicated operation process and is difficult to standardize the individualized preparation process, and requires high quality control.

### Summary

. In view of this, one of objectives of the present invention is to provide a B cell composition.

. To achieve the above objective, the technical solution of the present invention is:
. A B cell composition, wherein comprising an allogeneic B cell and an antigen of Epstein-Barr virus, the antigen is presented on membrane surface of the allogeneic B cell and combined with an antigen-presenting molecule on the membrane surface; the B cell composition is irradiated with ionizing irradiation lower than inactivation threshold.

. The team of the present invention uses an allogeneic B cell, which can effectively activate T cell immune response, and its activation effect is even better than that of an autologous B cell.

. Further, the allogeneic B cell retains biological activity but is incapable of proliferation after being irradiated with the ionizing irradiation.

. Further, the Epstein-Barr virus is inactivated after being irradiated with the ionizing irradiation.

. Further, the allogeneic B cell carries or does not carry live virus.

. Further, the ionizing irradiation is one or more of X-ray, γ ray, and Co⁶⁰ isotope.

. Further, the dose of the ionizing irradiation is about 10-200Gy.

. Further, the dose of the ionizing irradiation is about: 10Gy, 15Gy, 20Gy, 25Gy, 30Gy, 35Gy, 40Gy, 45Gy, 50Gy, 55Gy, 60Gy, 65Gy, 70Gy, 75Gy, 80Gy, 85Gy, 90Gy, 95Gy, 100Gy, 105Gy, 110Gy, 115Gy, 120Gy, 125Gy, 130Gy, 135Gy, 140Gy, 145Gy, 150Gy, 155Gy, 160Gy, 165Gy, 170Gy, 175Gy, 180Gy, 185Gy, 190Gy, 195Gy, or 200Gy.

. Further, dose rate of the ionizing irradiation is about 3-12Gy/min; irradiation time is about 200-600s.

. Further, the dose rate of the ionizing irradiation is about 3Gy/min, 3.5Gy/min, 4Gy/min, 4.5Gy/min, 5Gy/min, 5.5Gy/min, 6Gy/min, 6. 5 Gy/min, 7Gy /min, 7.5Gy/min, 8Gy/min, 8.5Gy/min, 9Gy/min, 9.5 Gy/min, 10Gy/min, 10.5Gy/min, 11 Gy/min, 11.5Gy/min or 12Gy /min; the irradiation time is about 200s, 225s, 250s, 275s, 300s, 325s, 350s, 375s, 400s, 425s, 450s, 475s, 500s, 525s, 550s, 575s or 600s.

. Further, the allogeneic B cell is subject to immortalization treatment and is capable of expanding for more than 40 generations; the immortalization treatment involves infecting the allogeneic B cell with the Epstein-Barr virus or the antigen of the Epstein-Barr virus.

. Further, the allogeneic B cell is a diploid cell.

. Further, the allogeneic B cell is subject to EBV infection treatment or EBV antigen loading.

. Further, the allogeneic B cell is cultured at 37°C, 5% CO₂ under serum-free or serum-containing condition before being irradiated with the ionizing irradiation

. Further, edge of the allogeneic B cell has an antenna-like protrusion.

. Further, the B cell composition also includes T cell.

. The objective of the present invention is also to provide a B cell vaccine.

. A B cell vaccine comprising the above B cell composition.

. Further, the B cell vaccine further comprises a coupling agent and/or an immune adjuvant. The coupling agent described in the present invention is a general medical sterile coupling agent, usually composed of carbomer, glycerol, purified water and other components. The immune adjuvant described in the present invention refers to an auxiliary substance that is injected into the body together with the antigen or in advance to enhance the capacity of immune response of the body to the antigen or to change the type of immune response. Currently, commonly used vaccine adjuvants can be classified into several categories such as aluminum salt adjuvants, protein-based adjuvants, nucleic acid-based adjuvants, lipid-containing adjuvants and mixed adjuvants according to their chemical composition.

. Further, dosage form of the B cell vaccine is subcutaneous injection, intradermal injection, local injection, intraperitoneal injection or intravenous injection.

. Further, the B cell vaccine is an allogeneic B cell vaccine. Of course, the present invention can also be prepared as an autologous B cell vaccine.

. Use of the above-mentioned B cell vaccine in the preparation of an anti-tumor drug, and the anti-tumor drug is used to treat a tumor carrying Epstein-Barr virus.

. Further, the tumor is related to EBV infection. Taking nasopharyngeal carcinoma as an example, studies have confirmed that DNAs and antigens of EBV can be detected in the cancer tissues of nasopharyngeal carcinoma patients, and EBV antibodies can be detected in their serum.

. Further, the tumor carrying Epstein-Barr virus comprises one or more of nasopharyngeal carcinoma, lymphoma, lethal midline granuloma (midline malignant reticulosis), infectious mononucleosis and gastric cancer, the lymphoma comprises Hodgkin's lymphoma (HD), non-Hodgkin's lymphoma (NHD), Burkitt's lymphoma (lymphoma in African Children), diffuse large B-cell lymphoma and post-transplant lymphoma.

. The objective of the present invention is also to provide a preparation method for a B cell vaccine.

. A preparation method for a B cell vaccine, comprising the following steps: 1) collecting a allogeneic B cell that is diploid, the allogeneic B cell is subject to immortalization treatment; 2) culturing and expanding the allogeneic B cell, the allogeneic B cell carries an EBV antigen; 3) irradiating the allogeneic B cells obtained in the step 2) with a certain dose (lower than inactivation threshold) of ionizing irradiation.

. Further, culture condition of the step 2) is: 37°C, 5% CO₂, without serum or with serum.

. Further, seeding density of the allogeneic B cell in the step 2) is 0.5-1 × 10⁶ cells/ml.

. Further, number of generation of expansion of the allogeneic B cell in the step 2) is greater than 100.

. Further, the ionizing irradiation in the step 3) is one or more of X-ray, γ ray, and Co ⁶⁰ isotope.

. Further, dose of the ionizing irradiation is about 10-200Gy.

. Further, the dose of the ionizing irradiation is about: 10Gy, 15Gy, 20Gy, 25Gy, 30Gy, 35Gy, 40Gy, 45Gy, 50Gy, 55Gy, 60Gy, 65Gy, 70Gy, 75Gy, 80Gy, 85Gy, 90Gy, 95Gy, 100Gy, 105Gy, 110Gy, 115Gy, 120Gy, 125Gy, 130Gy, 135Gy, 140Gy, 145Gy, 150Gy, 155Gy, 160Gy, 165Gy, 170Gy, 175Gy, 180Gy, 185Gy, 190Gy, 195Gy, or 200Gy.

. Further, dose rate of the ionizing irradiation is about 2-12Gy/min; irradiation time is about 200-600s.

. Further, the dose rate of the ionizing irradiation is about 3Gy/min, 3.5Gy/min, 4Gy/min, 4.5Gy/min, 5Gy/min, 5.5Gy/min, 6Gy/min, 6. 5 Gy/min, 7Gy /min, 7.5Gy/min, 8Gy/min, 8.5Gy/min, 9Gy/min, 9.5Gy /min, 10Gy/min, 10.5Gy/min, 11 Gy/min, 11.5Gy/min or 12Gy /min; the irradiation time is about 200s, 225s, 250s, 275s, 300s, 325s, 350s, 375s, 400s, 425s, 450s, 475s, 500s, 525s, 550s, 575s or 600s.

. Further, the allogeneic B cell used in the step 2) is a peripheral blood lymphocyte obtained from healthy people.

. Further, the allogeneic B cell is subjected to EBV infection before the step 2).

. The B cell vaccine prepared by the above preparation method.

. Further, the B cell vaccine also comprises a coupling agent and/or an immune adjuvant.

. Further, dosage form of the B cell vaccine is subcutaneous injection, intradermal injection, local injection, intraperitoneal injection or intravenous injection.

. Further, the B cell vaccine is capable of activating an immune cell in a human body, and the immune cell includes T cell.

. Further, the B cell vaccine up-regulates expression of related molecules involved in antigen presentation; the related molecules include CD36, SEC61A2, TAP1, SEC61A1, SEC61B or SEC61G.

. Further, the B cell vaccine is capable of specifically recognizing an Epstein-Barr virus antigen.

. Use of the above-mentioned B cell vaccine in the preparation of an anti-tumor drug, and the anti-tumor drug is used to treat tumors carrying Epstein-Barr virus.

. Further, the tumor carrying Epstein-Barr virus comprises one or more of nasopharyngeal carcinoma, lymphoma, lethal midline granuloma (midline malignant reticulosis), infectious mononucleosis and gastric cancer, the lymphoma comprises Hodgkin's lymphoma (HD), non-Hodgkin's lymphoma (NHD), Burkitt's lymphoma (lymphoma in African Children), diffuse large B-cell lymphoma and post-transplant lymphoma.

. The objective of the present invention is also to provide a method and system for improving B cell antigen presentation.

. A method for improving B cell antigen presentation, wherein comprising the following steps: (1) culturing and expanding a B cell, the B cell carries an EBV antigen; (2) irradiating the B cell obtained in the step (1) with a certain dose of ionizing irradiation.

. Further, the B cell is subject to immortalization treatment before the step (1).

. Further, the B cell retains biological activity but is incapable of proliferation after being irradiated with the ionizing irradiation.

. A system for improving B cell antigen presentation, wherein comprising a cell culture and expansion device for expanding B cell carrying an EBV antigen; and an irradiation device for irradiating the B cell with ionizing irradiation lower than inactivation threshold.

. Further, the system also includes: a cryopreservation device for cryopreserving the B cell irradiated with the ionizing irradiation.

. The objective of the present invention is also to provide a B cell composition, comprising: an allogeneic B cell and a virus antigen; the B cell composition is subject to a certain dose of ionizing irradiation.

. Further, the virus antigen is from human-susceptible virus, and the human-susceptible virus includes one or more of herpes virus, HIV virus and hepatitis virus.

. Further, the use of the B cell composition in the prevention or treatment of viral infection, the B cell composition has a specific antiviral effect.

### Beneficial effect of the present invention

. Aiming at the deficiencies of the tumor vaccines in the prior art, the present invention proposes a new B cell composition and a tumor vaccine based on the B cell composition, that is, an allogeneic B cell vaccine. The vaccine has the following characteristics or advantages:
. B cell has antigen presenting ability. The anti-tumor vaccine of the present invention uses allogeneic B cells as antigen presenting cells to present tumor-specific antigens or tumor-associated antigens. The use of allogeneic B cells to prepare anti-tumor vaccines is a new anti-tumor vaccine preparation strategy, which has good innovation and broad application prospects.

. The immune response activated by the anti-tumor vaccine of the present invention can specifically recognize the Epstein-Barr virus antigen, which only exists on tumor cells and does not exist in normal cells, so the vaccine has good specificity and high safety. Similarly, if the vaccine presents other tumor-specific antigens, it will also generate an immune response targeting other tumor types.

. B cells have clear antigen presentation ability and can efficiently present EBV antigen. In the present invention, B cells are required to be infected with Epstein-Barr virus to realize immortalization. In other words, the immortalized B cells, infected with EBV virus and capable of presenting EBV antigens, can be continuously expanded. Therefore, 100% of the vaccine cells (immortalized) produced by the present invention can present EBV antigens, which has obvious advantages.

. The safety of the vaccine of the present invention is good, which is specifically reflected in: (i) the B cells used to prepare the vaccine of the present invention are from peripheral blood lymphocytes which have normal (diploid) karyotype and have no tumorigenicity; (ii) immunization method of the vaccine of the present invention is subcutaneous or intradermal local immunization which has low risk of causing severe systemic non-anti-tumor immune response; (iii) in the process of preparing the vaccine of the present invention, the irradiated B cells retain biological activity but lose the ability to proliferate , and the Epstein-Barr virus is also inactivated.

. The allogeneic B cells used to prepare the vaccine of the present invention can be expanded to reach large quantities in vitro before being irradiated, which can realize standardized and large-scale preparation and ensure the quality and standard of vaccines used in clinical trials.

. To sum up, the present invention provides a new type of vaccine, which is prepared by using allogeneic B cells with normal karyotype. The vaccine of the present invention is allogeneic B cells after irradiation, most of which are living cells (that is, maintain activity), hardly proliferate, can stimulate anti-tumor cell immune response, have no tumorigenicity, and have high safety. In addition, because the allogeneic B cells used in the present invention can be expanded to reach large quantities in vitro before irradiation, and can efficiently activate anti-tumor immune responses in patients after irradiation, the preparation process can be standardized, scaled up, and easier to ensure the quality and standard of vaccines used in clinical trials.

### Description of Drawings

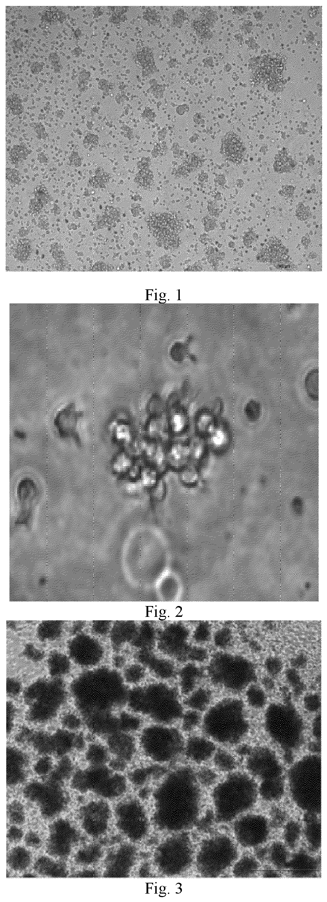

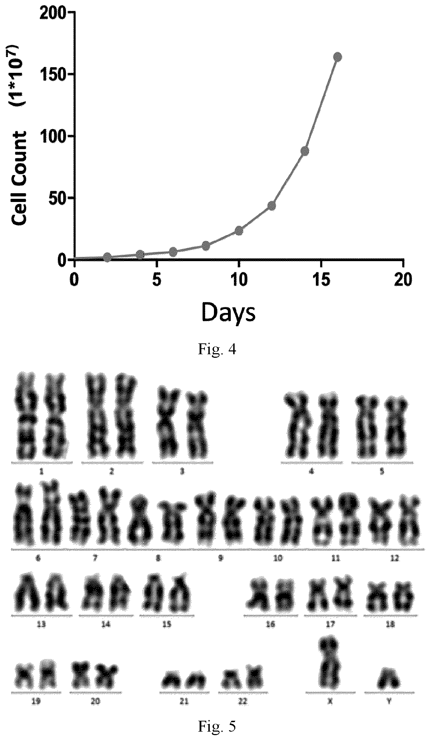

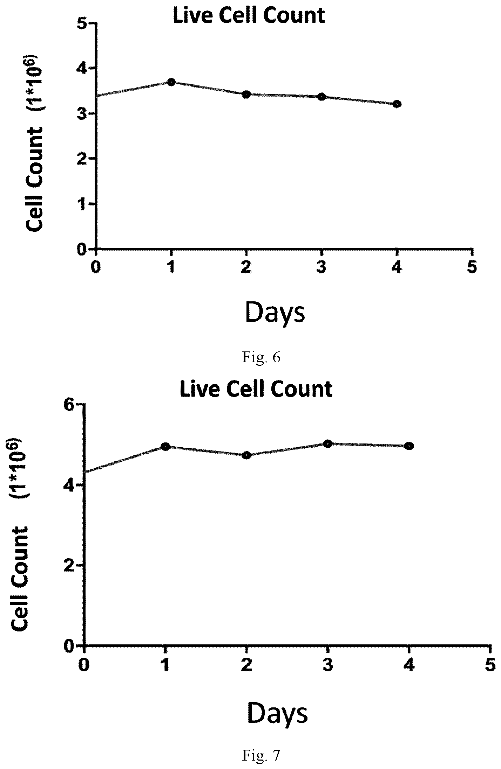

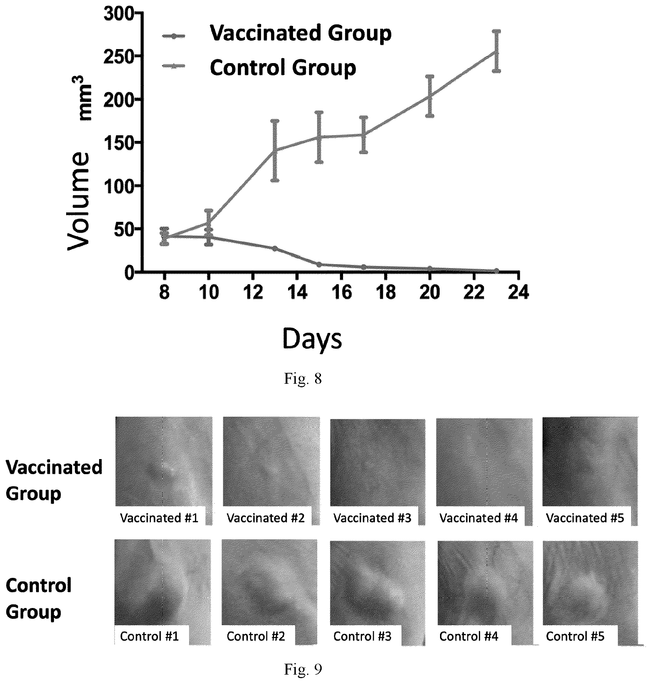

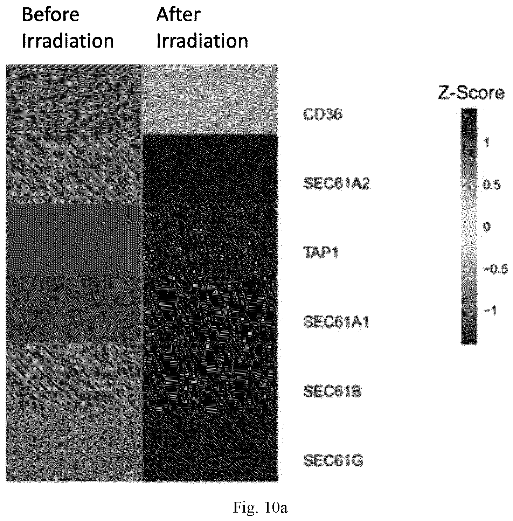

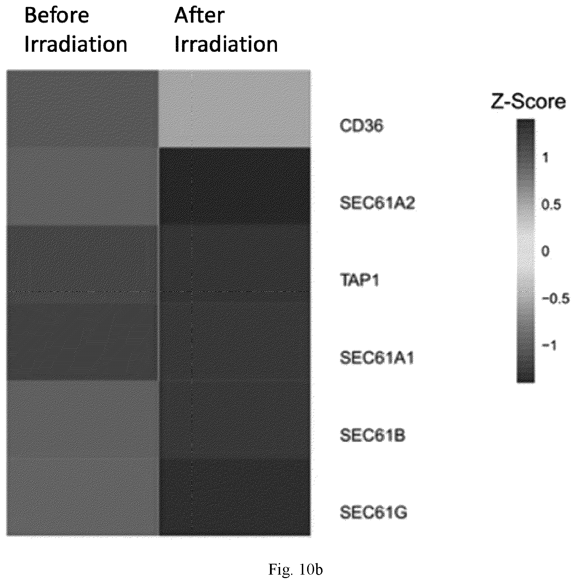

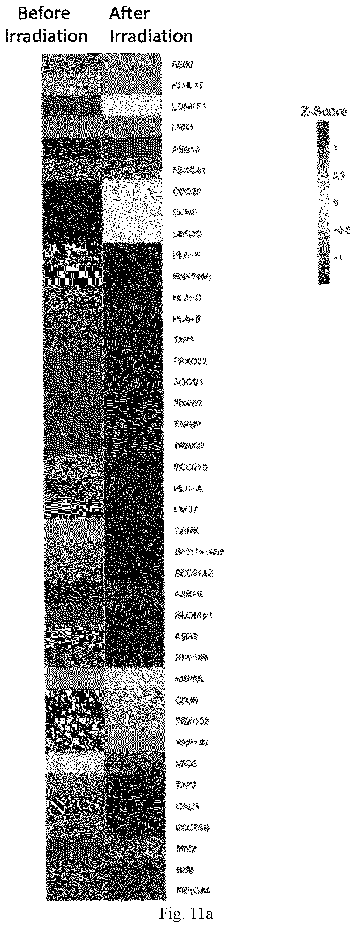

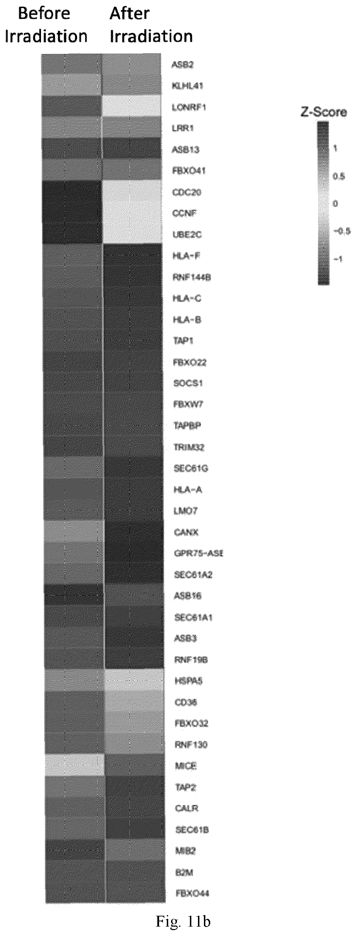

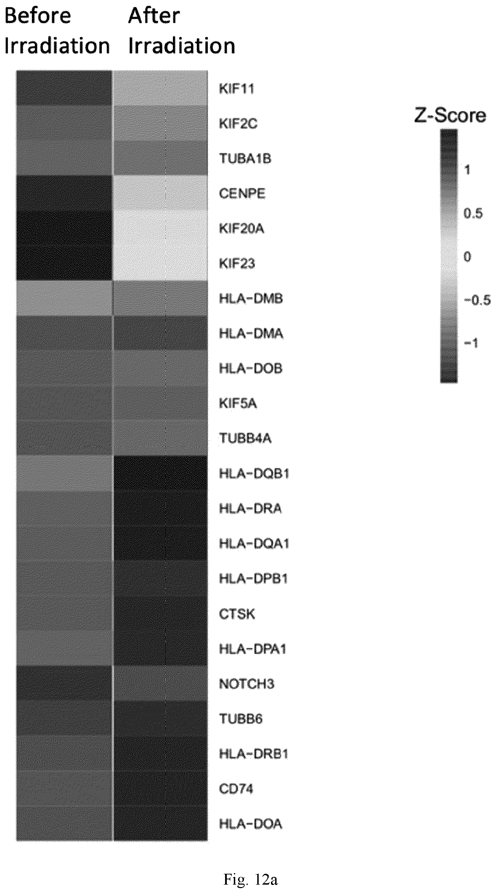

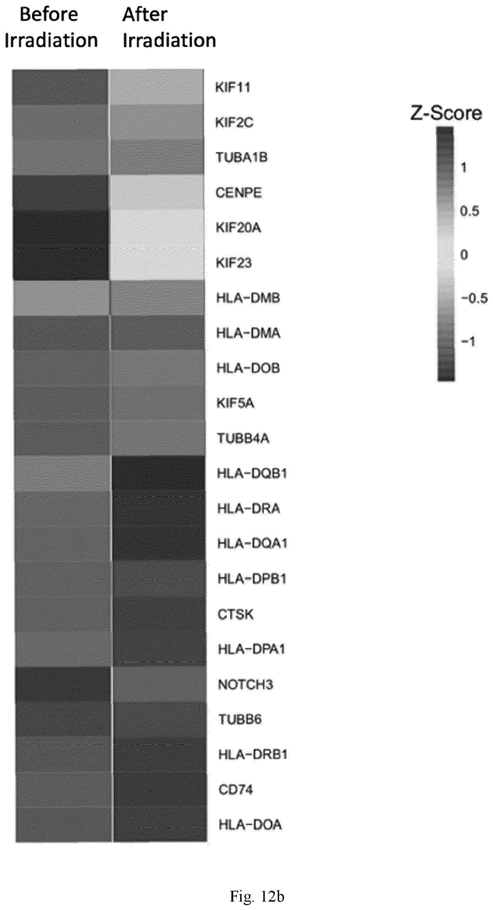

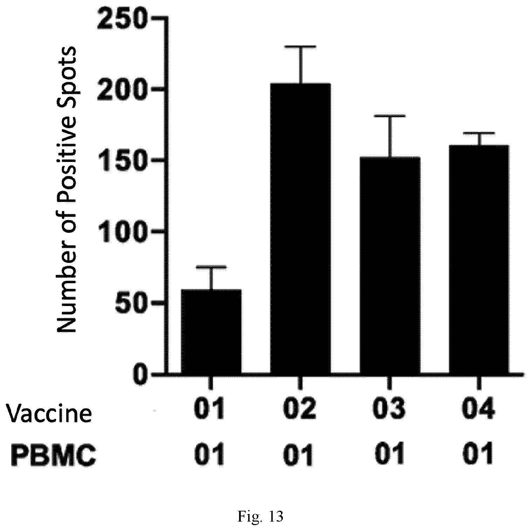

. To make the embodiments of the present invention or the technical solutions in the prior art clearer, the drawings required to be used in the description of the embodiments or the prior art will be briefly introduced below. It is obvious that the drawings described below are some embodiments of the present invention, and that other drawings can be obtained from these drawings for those of ordinary skill in the art without making inventive effort.
. Fig. 1 shows a microscopic view of B cells in normal culture.
. Fig. 2 shows a microscopic view of B cells in normal culture.
. Fig. 3 shows a microscopic view of B cells in optimized culture.
. Fig. 4 shows a statistical plot of B cells in optimized culture (horizontal coordinate: time; vertical coordinate: number of cells).
. Fig. 5 shows a karyotype diagram of B cells.
. Fig. 6 shows a statistical plot of live cells after irradiation (horizontal coordinate: time; vertical coordinate: number of cells).
. Fig. 7 shows a statistical plot of total cells after irradiation (horizontal coordinate: time; vertical coordinate: number of cells).
. Fig. 8 shows a statistical plot of antitumor effect of the vaccine (horizontal coordinate: time; vertical coordinate: tumor size).
. Fig. 9 shows photographs of antitumor effect of the vaccine in the mouse model.
. Fig. 10a shows significant changes in APC cross-presentation-related molecules in vaccine cells after irradiation.
. Fig. 10b shows significant changes in APC cross-presentation-related molecules in vaccine cells after irradiation.
. Fig. 11a shows significant changes in endogenous presentation-related molecules in vaccine cells after irradiation; Fig. 11b shows significant changes in endogenous presentation-related molecules in vaccine cells after irradiation.
. Fig. 12a shows significant changes in exogenous presentation-related molecules in vaccine cells after irradiation; Fig. 12b shows significant changes in exogenous presentation-related molecules in vaccine cells after irradiation.
. Fig. 13 shows that vaccine cells prepared on the basis of allogeneic B cells are more efficient in activating immune cells.

### Detailed Description

. To make the objective, the technical solutions and advantages of the embodiments of the present invention clearer, the technical solutions of the embodiments of the present invention will be clearly and completely described below in combination with drawings. It is obvious that the described embodiments are some of the embodiments of the present invention, not all of the embodiments. Based on the embodiments of the present invention, all other embodiments obtained by those of ordinary skill in the art without making inventive effort shall belong to the protection scope of the present invention.

. The embodiments are for the purpose of better illustration of the present invention, but the contents of the present invention are not limited to the embodiments. Therefore, non-essential improvements and adjustments of the embodiments made by those skilled in the art according to the above-mentioned contents of the present invention still belong to the protection scope of the present invention.

. Unless otherwise specified, the term "include" and "comprise" and their grammatical variations thereof are used to indicate "open-ended" or "inclusive" language, such that they include enumerated technical features but also allow for the inclusion of additional technical features that are not enumerated.

. As used herein, the term "about" (for example, used for indicating dose rate of ionizing irradiation or irradiation time values), typically means +/-5% of the stated value, more typically +/-4% of the stated value, more typically +/-3% of the stated value, more typically, +/-2% of the stated value, even more typically +/-1% of the stated value, and even more typically +/-0.5% of the stated value.

. Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

. Cell immortalization in the present invention refers to a process in which cells are cultured in vitro using genetic changes or various external stimulating factors to avoid the normal senescence and death processes of cells, thereby achieving long-term subculture and infinite division and proliferation. The currently used methods of cell immortalization include the use of radioactive elements, telomere-telomerase activation, and viral gene transfection, etc.

. An allogeneic B cell in the present invention refers to a B cell derived from different individuals of humans or the same species of animals. For example, the B cells described in the present invention are peripheral blood B cells derived from different healthy people. The experimental results of the present invention show that the vaccine derived from allogeneic B cells from different people has the same good safety and anti-tumor immune response as vaccines derived from autologous B cells of a patient, but the vaccine in the present invention has more ways to obtain and is more convenient to prepare.

### Embodiment 1

. The characteristics of the vaccine cells of the present invention are as follows: the vaccine is irradiated B cells, most of which are living cells, hardly proliferate, but can stimulate anti-tumor cell immune response.

. Before irradiation, the vaccine cells are immortalized diploid B cells under serum-free culture conditions (normal culture condition is under 20% serum), which can be expanded to reach large quantities and can present EBV and other antigens.

. The B cells used to prepare the vaccine of the present invention can carry EBV antigens in various ways: (i) B cells are the natural host of EBV, if contacting EBV, B cells are infected with EBV which can also make B cells achieve immortalization; (ii) B cells can also be genetically engineered to express specific EBV antigens.

. Before irradiation, B cells were cultured at 37°C, 5% CO₂ under serum-free culture conditions with a cell seeding density of 0.5 ~ 1×10⁶ cells/ml. B cells mainly grew in clusters in suspension, with a small part grew in a single scattered state. Antenna-like protrusions can be seen on the edge of B cells. See Fig. 1 and Fig. 2 for details.

. B cells are cultured according to the above-mentioned optimized culture conditions provided by the present invention. After culture, the B cells are in a more obvious clonal growth, and can be expanded by more than 100 folds in two weeks. See Fig. 3 and Fig. 4 for details. Fig. 4 is a statistical plot of optimized culture of B cells. After 16 days of culture, the number of cells increased significantly. See Table 1 for details.

.

**Table 1 Statistical table of the number of B cells**

| Culture time (days) | Cell number (10⁷) |
|---|---|
| 0 | 1.4 |
| 2 | 2 |
| 4 | 4.2 |
| 6 | 6.4 |
| 8 | 11.47 |
| 10 | 23.57 |
| 12 | 43.76 |
| 14 | 87.9 |
| 16 | 163.9 |

. Please note that according to actual needs, the culture conditions of B cells before irradiation can also contain a certain amount of serum. In addition, the expansion fold of B cells can also be adjusted, for example, the expansion of more than 40 folds can be followed by irradiation treatment.

### Embodiment 2

. Karyotype analysis of B cells for the preparation of the vaccine of the present invention: take cultured B cells (expansion > 40 generations, preferably expansion > 100 generations) on a glass slide to prepare observation slices, digest with trypsin for 2-3 minutes, and rinse with 0.9% normal saline to stop the enzymatic action of trypsin, then stain with Giemsa stain solution for 10-15 minutes, rinse and dry the slides, and perform karyotype analysis under a microscope to observe whether there are any abnormalities in their morphology. The analyzed B cells were of normal diploid karyotype, and the karyotype of the cells submitted for inspection was: 46, XY. See Fig. 5 for details. In addition, from the perspective of principle action of the vaccine, B cells with a karyotype of 46, XX also have similar immune activation capabilities and can also be used to make vaccine cells of the present invention.

### . Embodiment 3

. Virus detection of vaccine cells of the present invention: use cell DNA extraction kit to extract B cell DNA or RNA, use real-time fluorescence quantitative PCR to perform hepatitis B virus (HBV-DNA) quantitative detection, hepatitis C virus (HCV-RNA) quantitative detection, human Papillomavirus (HPV16/18-DNA) qualitative detection, human papillomavirus (HPV6/11-DNA) qualitative detection, parvovirus (B19-DNA) qualitative detection, cytomegalovirus (CMV-DNA) quantitative detection and Epstein-Barr virus (EB DNA) quantitative detection. The results showed that the quantitative detection result of HBV-DNA in B cells was <5.00E+02 IU/mL, which was lower than the lowest limit of detection of 5.00E+02 IU/mL; the quantitative detection result of HCV-RNA was <5.00E+02 IU/mL, which was lower than the lowest limit of detection of 5.00E+02 IU/mL; the qualitative detection result of human papillomavirus (HPV16/18-DNA) was negative (-); the qualitative test detection of human papillomavirus (HPV6/11-DNA) was negative (-); the qualitative detection result of parvovirus (B19-DNA) was negative (-); the quantitative detection result of cytomegalovirus (CMV-DNA) was <1.00E+03 IU/mL, which was lower than the lowest limit of detection of 1.00E+03 IU/mL; the quantitative detection result of Epstein-Barr virus (EB DNA) was 6.17E+06 Copies/mL.

. The above detection results show that the B cells used to prepare the present invention do not carry other viruses except EBV

### . Embodiment 4

. Preparation of vaccine cells by irradiation, and the characteristics of vaccine cells after irradiation:
. After suspension in the serum-free medium according to the above method, B cells were irradiated using the irradiation instrument RS-2000 Biological Irradiator, and the irradiation conditions were as follows:
. Irradiation dose: Irradiation was carried out at about 10-200Gy. Specifically, the irradiation dose can be chosen from about 10Gy, 15Gy, 20Gy, 25Gy, 30Gy, 35Gy, 40Gy, 45Gy, 50Gy, 55Gy, 60Gy, 65Gy, 70Gy, 75Gy, 80Gy, 85Gy, 90Gy, 95Gy, 100Gy, 105Gy, 110Gy, 115Gy 120Gy, 125Gy, 130Gy, 135Gy, 140Gy, 145Gy, 150Gy, 155Gy, 160Gy, 165Gy, 170Gy, 175Gy, 180Gy, 185Gy, 190Gy, 195Gy, or 200Gy.
. Irradiation dose rate: about 3-12Gy/min. Specifically, the irradiation dose rate is about 3Gy/min, 3.5Gy/min, 4Gy/min, 4.5Gy/min, 5Gy/min, 5.5Gy/min, 6Gy/min, 6.5Gy/min, 7Gy/min, 7.5Gy/min, 8Gy/min, 8. 5 Gy/min, 9Gy/min, 9.5Gy/min, 10Gy/min, 10.5Gy/min, 11 Gy/min, 11.5Gy/min, or 12Gy/min.
. Irradiation time: about 200-600s. Specifically, the irradiation time is about 200 s, 225 s, 250 s, 275 s, 300 s, 325 s, 350 s, 375 s, 400 s, 425 s, 450 s, 475 s, 500 s, 525 s, 550 s, 575 s, or 600 s.

. Energy for irradiation: 160KV (kilovoltage); current intensity for irradiation: 25mA.

. Other conditions for irradiation: no filter, adding reflector.

. The morphology of B cells was normal after irradiation, and the living cells accounted for about 80%. The number of living cells and the total number of cells remained unchanged after 4-5 days of continuous culture, indicating that B cells had no obvious proliferation ability after irradiation. See Fig. 6 and Fig. 7 for details. Among them, Fig. 6 is a statistical plot of live cells after irradiation, and the specific values are shown in Table 2. Fig. 7 is a statistical plot of total cells after irradiation, and the specific values are shown in Table 3.

.

**Table 2 Statistical table of living cells**

| Culture time (days) | Cell number (10⁶ ) |
|---|---|
| 0 | 3.38 |
| 1 | 3.69 |
| 2 | 3.42 |
| 3 | 3.37 |
| 4 | 3.21 |

**Table 3 Statistical table of total cell number**

| Culture time (days) | Cell number (10⁶ ) |
|---|---|
| 0 | 4.3 |
| 1 | 4.95 |
| 2 | 4.73 |
| 3 | 5.02 |
| 4 | 4.96 |

. B cells used to prepare the vaccine were irradiated at least once according to the above conditions before cryopreservation. During cryopreservation, cryopreservation solution (BioLife Sulution, USA) was used to freeze vaccine cells (i.e. irradiated cells) at 1×10⁷ cells/ml.

### . Embodiment 5

. Tumorigenicity assay of vaccine cells: 6-8 weeks old, BALB/c nude mice, females, 10, were used for the experiments, kept in a standard SPF-level animal room, with room temperature maintained at 25°C and free access to food and water during the experiments. After irradiation, vaccine cells were washed twice with PBS and resuspended in serum-free culture medium to a final concentration of 10⁸/ml. BALB/c nude mice were inoculated subcutaneously with 100 ul of vaccine cell suspension of 1×10⁷ cells/ml. After subcutaneous inoculation of vaccine cells, nude mice were observed weekly for 9 weeks for survival and for tumor formation at the inoculation site. No nude mice had subcutaneous xenograft tumor formation, indicating that the present vaccine cells were non-tumorigenic.

### . Embodiment 6

. Antitumor effect: well-grown human B cells carrying EBV antigens were taken and irradiated to prepare vaccine cells according to the above experimental parameters, and then the vaccine cells were resuspended with serum-free culture medium to a final concentration of 5×10⁷/ml, and 200 ul of vaccine cell suspension (1×10⁷ cells) were subcutaneously injected into the groin of BALB/c mice (6-8 weeks old, female) at multiple points for 1, 2, and 4 weeks, respectively. At week 5, BALB/c mice that had been immunized three times were executed, and the spleens were removed after aseptic treatment. Splenic lymphocytes were isolated and resuspended in RPMI-1640 medium at a concentration of 10⁸/ml. 150 ul of spleen lymphocyte suspension (containing 1.5×10⁷ lymphocytes) was injected via tail vein into tumor-bearing mice that had been inoculated with nasopharyngeal carcinoma cells C666-1 (EBV+) for three injections. Tumors were measured every two days, tumor volumes were calculated, and tumor growth curves were plotted.

. The results showed that the tumor growth of mice in the adoptive immune cell therapy group was significantly slower than that of the control group and began to gradually decrease on day 10 after inoculation, and all tumors in the treatment group regressed on day 20. The details are shown in Figs. 8 and 9. Fig. 8 shows a statistical plot of the anti-tumor effect of the vaccine, and the specific values are shown in Table 4.

.

**Table 4 Statistical table of anti-tumor effect of the vaccine**

| time (day) | Tumor volume of experimental group (mm³) | | | | | | Tumor volume of control group (mm³) | | | | |
|---|---|---|---|---|---|---|---|---|---|---|---|
| | Individu al 1 | Individu al 2 | Individu al 3 | Individu al 4 | Individu al 5 | Individu al 6 | Individu al 1 | Individu al 2 | Individu al 3 | Individu al 4 | Individu al 5 |
| 8 | 77.15 | 15.32 | 26.64 | 54.32 | 32.81 | 41.43 | 51.60 | 44.80 | 50.10 | 27.74 | 20.18 |
| 10 | 72.90 | 15.74 | 22.57 | 55.94 | 34.33 | 40.79 | 99.39 | 66.93 | 67.59 | 31.59 | 18.78 |
| 13 | 26.02 | 33.28 | 25.13 | 28.68 | 24.38 | 25.47 | 219.16 | 174.26 | 193.47 | 58.75 | 57.02 |
| 15 | 13.62 | 11.64 | 8.29 | 5.24 | 6.66 | 7.98 | 195.31 | 200.34 | 207.06 | 115.14 | 61.71 |
| 17 | 9.16 | 8.72 | 8.09 | 4.56 | 2.35 | 2.96 | 201.03 | 180.91 | 190.08 | 123.51 | 98.17 |
| 20 | 4.22 | 2.75 | 8.59 | 2.14 | 3.08 | 2.96 | 205.20 | 254.53 | 253.37 | 158.41 | 146.12 |
| 23 | 1.08 | 5.01 | 1.00 | 1.10 | 0.77 | 0.75 | 299.22 | 317.22 | 248.69 | 216.17 | 196.75 |

. Please note that the above experiment is different from using vaccine cells to treat cancer patients. In order to test whether human immune cells can activate mouse T cells in the mouse model, and then kill human cancer cells in the mouse model, the vaccine cells of the present invention were first injected into BALB/c mice with a healthy immune system to activate T cells in the BALB/c mice, and then the activated T cells were injected into tumor-bearing mice (with a flawed immune system) that have been inoculated with human tumor cells. In the treatment of a cancer patient with a healthy immune system, the vaccine cells can be injected directly into the patient's body to activate T cells in the patient's body, and then eliminate the tumor/cancer cells (in addition to nasopharyngeal carcinoma, it also includes : various lymphomas (Hodgkin's lymphoma (HD), non-Hodgkin's lymphoma (NHD), Burkitt's lymphoma (lymphoma in African children), diffuse large B-cell lymphoma and post-transplant lymphoma, etc.), lethal midline granuloma (midline malignant reticulosis), infectious mononucleosis, and gastric cancers) cells carrying EBV virus antigens.

. In addition, the above experiment based on the mouse model also shows that although the vaccine cells of the present invention come from human, they can still activate the immune system of mice - in other words, the vaccine cells of the present invention can function in heterogeneous organisms, then they should also be possible to activate and have an effect in the immune system of allogeneic individuals (different individuals of same species (Homo sapiens)).

. Fig. 13 further shows that vaccine cells prepared based on allogeneic B cells can not only activate T cells in the allogeneic individuals, but even have a better effect - in the experiments in Fig. 13 , the B cells used to prepare vaccine cells were taken from individual 01, after co-cultivating vaccine cells from 01 with mononuclear cells (PBMC) or lymphocytes (PBL) in the peripheral blood of individuals 02, 03, and 04, the number of activated lymphocytes was significantly higher than that activated in individual 01. See Table 5 for values of positive spots of individuals vaccinated in Fig. 13.

.

**Table 5 Number of positive spots**

| Individual | number of spots | | |
|---|---|---|---|
| 01 | 45 | 76 | 58 |
| 02 | 187 | 191 | 234 |
| 03 | 127 | 146 | 184 |
| 04 | 163 | 152 | 168 |

### . Embodiment 7

### . Antigen presentation assay of irradiated B cells

. Divide the vaccine cells being cultured into 2 parts, one part of 5×10⁶ vaccine cells was centrifuged at 600g for 5 minutes, the supernatant was discarded, then resuspended in 1ml of ice TRIzol, repeated pipetting until the liquid became clear, and then frozen at -80°C. The other one contained 1.5×10⁷ vaccine cells. After γ-ray irradiation, it's divided into 3 parts on average. Each part contained 5×10⁶ vaccine cells, and continued to culture in the incubator, and centrifuged at 600g for 5 minutes after 6 hours, 12 hours and 24 hours respectively. The supernatant was discarded, and 1ml of ice TRIzol was added to resuspend vaccine cells. Perform repeated pipetting until the liquid became clear, and then freeze them at -80°C. The results of RNA-Seq of next-generation high-throughput sequencing of vaccine cells (transport on dry ice) are as follows:
1) Analyze the changes of B cell transcriptome before and after irradiation by RNA-seq of high-throughput transcriptome sequencing. The related molecules of B cells involved in antigen presentation after irradiation (for example, CD36, SEC61A2, TAP1, SEC61A1, SEC61B, SEC61G) expression levels were up-regulated: except for CD36, the expression levels of other molecules showed up-regulation at the measurement points of 6 hours and 12 hours, and showed a down-regulation trend at the measurement point of 24 hours; expression levels of CD36 showed a trend of gradual up-regulation at the measurement points of 6 hours, 12 hours and 24 hours (Fig. 10).
2) Flow cytometry detection of changes of MHC-I, MHC-II, CD80, CD86, CD40, CD54 and other antigen presentation-related molecules on the surface of B cells before and after irradiation (Fig. 11, Fig. 12). In Fig. 11, the expression of ASB2-UBE2C molecule showed a downward trend over time; the expression of HLA-F-FBXO44 molecule showed an upward trend over time. In Fig. 12, the expression of KIF11-KIF23 molecule showed a downward trend (but with fluctuations) over time, and the expression of HLA-DMB-HLA-DOA molecule showed an upward trend over time (but the expression level at the measurement point of 24 hours was lower than that at the measurement point of 12 hours).
3) The expression of intracellular cytokines such as Granzyme B, IFN-γ, and IL2 of T cells educated by B cells after irradiation detected by Flow cytometry indirectly proves that the function of the educated CTLs (Cytotoxic T Cells).
4) As shown in Fig. 13, the Elispot assay of the IFN-γ secretion of T cells indirectly proves that the function of the educated CTLs.

. The above experimental results show that after irradiation, the antigen presentation ability of B cells is improved. Therefore, the present invention can be used to efficiently present not only EBV virus antigens, but also can efficiently present antigens of viruses such as human herpes virus, HIV virus, and hepatitis virus to prepare a B cell-virus antigen composition to activate the immune response against the corresponding virus in vivo and produce a specific antiviral effect.

. B cells have the ability to present antigens, and can present EBV antigens to the cell surface, and exist on the cell membrane in the form of antigen peptide-HLA complexes combined with HLA (Human Leukocyte Antigen) molecules. Although existing studies have shown that B cells transformed by EBV virus can activate T cells in vitro and educate T cells to become CTL cells that can specifically recognize EBV antigens which can produce obvious anti-tumor effects after expansion to reach large quantities and infusion back into tumor patients. However, it is difficult to promote and apply in clinical practice because of cumbersome operation, long cycle and high cost in this process.

. For example, adoptive infusion therapy of CTLs targeting EBV antigens in the clinical trial stage in the prior art includes the in vitro preparation of T lymphocytes (LCL-CTL) that can recognize EBV antigens and then infusion back of the LCL-CTL cells to the patient for treatment. In some clinical trials, the use of LCL-CTL has produced good therapeutic effects, achieving 30-50% complete recover (CR) for relapsed and refractory lymphoma. However, the disadvantages of this treatment are also very obvious: (i) due to infusion back of T cells that are activated in vitro, the whole process takes a long time, and many patients cannot wait for such a long time or lose the therapeutic window; (ii) due to kill EBV-infected cancer cells by infusion back of LCL-CTL, rejection can only be prevented by individualized cell preparation and treatment, and multiple rounds of stimulation are required to generate a sufficient number of CTL cells, which is technically cumbersome; (iii) CTL cells from some patients cannot grow sufficiently to complete the treatment.

. The innovation of the present invention is to use irradiated B cells as a vaccine to immunize patients, and exert the antigen presentation ability of B cells in the human body, thereby activating the immune response in vivo and producing anti-tumor effects - this avoids the problem of long cycle of in vitro culture, education, and expansion of T cells.

. In addition, although individualized therapy (that is, using the patient's own B cells to prepare the vaccine) can be adopted in the present invention, it is also technically feasible to use allogeneic B cells to prepare the vaccine to educate T cells in the patient's body -because the T cells are educated in vivo, human body will naturally not reject the activated T cells in vivo; while the allogeneic B cells may be more conducive to the activation of the human immune system. In fact, in the present invention, allogeneic B cells have a better effect on educating T cells, and will produce better anti-tumor effects. Therefore, the present invention can also be prepared into a standardized cell line, which may have better therapeutic effect, and be feasible to large-scale production.

. It should be noted that the term "include", "comprise" or any variant thereof is intended to encompass nonexclusive inclusion so that a process, method, article or device including a series of elements includes not only those elements but also other elements which have been not listed definitely or an element(s) inherent to the process, method, article or device. Moreover, the expression "comprising a(n) ... " in which an element is defined will not preclude presence of an additional identical element(s) in a process, method, article or device comprising the defined element(s) unless further defined.

The embodiments of the present invention are described above with reference to the accompanying drawings, but the present invention is not limited to the aforementioned specific embodiments. The aforementioned embodiments are merely illustrative and not limiting. For those of ordinary skill in the art, many forms can be made under the teaching of present invention without departing from the spirit of the present invention and the scope of the claims, all of which shall fall within the protection scope of the present invention.

## Claims

1. A B cell composition, wherein comprising an allogeneic B cell and an antigen of Epstein-Barr virus, the antigen is presented on membrane surface of the allogeneic B cell and combined with an antigen-presenting molecule on the membrane surface; the B cell composition is irradiated with a certain dose of ionizing irradiation.

2. The B cell composition according to claim 1, wherein the allogeneic B cell retains biological activity but is incapable of proliferation after being irradiated with the ionizing irradiation.

3. The B cell composition according to claim 1, wherein the Epstein-Barr virus is inactivated after being irradiated with the ionizing irradiation.

4. The B cell composition according to claim 1, wherein the allogeneic B cell carries or does not carry live virus.

5. The B cell composition according to claim 1, wherein the ionizing irradiation is one or more of X-ray, γ ray, and Co⁶⁰ isotope.

6. The B cell composition according to claim 1, wherein the dose of the ionizing irradiation is about 10-200Gy.

7. The B cell composition according to claim 7, wherein the dose of the ionizing irradiation is about: 10Gy, 15Gy, 20Gy, 25Gy, 30Gy, 35Gy, 40Gy, 45Gy, 50Gy, 55Gy, 60Gy, 65Gy, 70Gy, 75Gy, 80Gy, 85Gy, 90Gy, 95Gy, 100Gy, 105Gy, 110Gy, 115Gy, 120Gy, 125Gy, 130Gy, 135Gy, 140Gy, 145Gy, 150Gy, 155Gy, 160Gy, 165Gy, 170Gy, 175Gy, 180Gy, 185Gy, 190Gy, 195Gy, or 200Gy.

8. The B cell composition according to claim 1, wherein dose rate of the ionizing irradiation is about 3-12Gy/min; irradiation time is about 200-600s.

9. The B cell composition according to claim 8, wherein the dose rate of the ionizing irradiation is about 3Gy/min, 3.5Gy/min, 4Gy/min, 4.5Gy/min, 5Gy/min, 5. 5 Gy/min, 6Gy/min, 6.5Gy/min, 7Gy/min, 7.5Gy/min, 8Gy/min, 8. 5 Gy/min, 9Gy/min, 9.5Gy/min, 10Gy/min, 10.5Gy/min, 11 Gy/min, 11.5Gy/min or 12Gy/min; the irradiation time is about 200s, 225s, 250s, 275s, 300s, 325s, 350s, 375s, 400s, 425s, 450s, 475s, 500s, 525s, 550s, 575s or 600s.

10. The B cell composition according to claim 1, wherein the allogeneic B cell is subject to immortalization treatment and is capable of expanding for more than 40 generations; the immortalization treatment involves infecting the allogeneic B cell with the Epstein-Barr virus or the antigen of the Epstein-Barr virus.

11. The B cell composition according to claim 10, wherein the allogeneic B cell is a diploid cell.

12. The B cell composition according to claim 11, wherein the allogeneic B cell is subject to EBV infection treatment or EBV antigen loading.

13. The B cell composition according to claim 12, wherein the allogeneic B cell is cultured at 37°C, 5% CO₂ under serum-free or serum-containing condition before being irradiated with the ionizing irradiation

14. The B cell composition according to claim 13, wherein edge of the allogeneic B cell has an antenna-like protrusion.

15. A B cell vaccine, wherein comprising the B cell composition according to claim 1.

16. The B cell vaccine according to claim 15, wherein further comprising a coupling agent and/or an immune adjuvant.

17. The B cell vaccine according to claim 15, wherein dosage form of the B cell vaccine is subcutaneous injection, intradermal injection, local injection, intraperitoneal injection or intravenous injection.

18. Use of the B cell vaccine according to claim 15 in the preparation of an anti-tumor drug, wherein the anti-tumor drug is used to treat a tumor carrying Epstein-Barr virus.

19. The application of the B cell vaccine in the preparation of an antitumor drug according to claim 18, wherein the tumor carrying Epstein-Barr virus comprises one or more of nasopharyngeal carcinoma, lymphoma, lethal midline granuloma (midline malignant reticulosis), infectious mononucleosis and gastric cancer, the lymphoma comprises Hodgkin's lymphoma (HD), non-Hodgkin's lymphoma (NHD), Burkitt's lymphoma (lymphoma in African Children), diffuse large B-cell lymphoma and post-transplant lymphoma.

20. A preparation method for a B cell vaccine, wherein comprising the following steps:
(1) collecting a allogeneic B cell that is diploid, the allogeneic B cell is subject to immortalization treatment;
(2) culturing and expanding the allogeneic B cell, the allogeneic B cell carries an EBV antigen;
(3) irradiating the allogeneic B cells obtained in the step (2) with ionizing irradiation lower than inactivation threshold.

21. The preparation method according to claim 20, wherein culture condition of the step (2) is: 37°C, 5% CO₂, without serum or with serum.

22. The preparation method according to claim 20, wherein seeding density of the allogeneic B cell in the step (2) is 0.5-1 × 10⁶ cells/ml.

23. The preparation method according to claim 20, wherein number of generation of expansion of the allogeneic B cell in the step (2) is greater than 100.

24. The preparation method according to claim 20, wherein the ionizing irradiation in the step (3) is one or more of X-ray, γ ray, and Co⁶⁰ isotope.

25. The preparation method according to claim 20, wherein dose of the ionizing irradiation in the step (3) is about 10-200Gy.

26. The preparation method according to claim 25, wherein the dose of the ionizing irradiation is about: 10Gy, 15Gy, 20Gy, 25Gy, 30Gy, 35Gy, 40Gy, 45Gy, 50Gy, 55Gy, 60Gy, 65Gy, 70Gy, 75Gy, 80Gy, 85Gy, 90Gy, 95Gy, 100Gy, 105Gy, 110Gy, 115Gy, 120Gy, 125Gy, 130Gy, 135Gy, 140Gy, 145Gy, 150Gy, 155Gy, 160Gy, 165Gy, 170Gy, 175Gy, 180Gy, 185Gy, 190Gy, 195Gy, or 200Gy.

27. The preparation method according to claim 20, wherein dose rate of the ionizing irradiation is about 2-12Gy/min; irradiation time is about 200-600s.

28. The preparation method according to claim 27, wherein the dose rate of the ionizing irradiation is about 3Gy/min, 3.5Gy/min, 4Gy/min, 4.5Gy/min, 5Gy/min, 5. 5 Gy/min, 6Gy/min, 6.5Gy/min, 7Gy/min, 7.5Gy/min, 8Gy/min, 8. 5 Gy/min, 9Gy/min, 9.5Gy/min, 10Gy/min, 10.5Gy/min, 11 Gy/min, 11.5Gy/min or 12Gy/min; the irradiation time is about 200s, 225s, 250s, 275s, 300s, 325s, 350s, 375s, 400s, 425s, 450s, 475s, 500s, 525s, 550s, 575s or 600s.

29. The preparation method according to claim 20, wherein the allogeneic B cell used in the step (2) is a peripheral blood lymphocyte obtained from healthy people.

30. The preparation method according to claim 29, wherein the allogeneic B cell is subjected to EBV infection before the step (2).

31. The B cell vaccine prepared by the preparation method according to any one of claims 20-30.

32. The B cell vaccine according to claim 31, wherein the B cell vaccine also comprises a coupling agent and/or an immune adjuvant.

33. The B cell vaccine according to claim 32, wherein dosage form of the B cell vaccine is subcutaneous injection, intradermal injection, local injection, intraperitoneal injection or intravenous injection.

34. The B cell vaccine according to claim 31, wherein the B cell vaccine is capable of activating an immune cell in a human body, and the immune cell includes T cell.

35. The B cell vaccine according to claim 31, wherein the B cell vaccine up-regulates expression of related molecules involved in antigen presentation; the related molecules include CD36, SEC61A2, TAP1, SEC61A1, SEC61B or SEC61G.

36. The B cell vaccine according to claim 31, wherein the B cell vaccine is capable of specifically recognizing an Epstein-Barr virus antigen.

37. Use of the B cell vaccine according to claim 31 in the preparation of an anti-tumor drug, wherein the anti-tumor drug is used to treat a tumor carrying Epstein-Barr virus.

38. The use of the B cell vaccine in the preparation of an anti-tumor drug according to claim 37, wherein the tumor carrying Epstein-Barr virus comprises one or more of nasopharyngeal carcinoma, lymphoma, lethal midline granuloma (midline malignant reticulosis), infectious mononucleosis and gastric cancer, the lymphoma comprises Hodgkin's lymphoma (HD), non-Hodgkin's lymphoma (NHD), Burkitt's lymphoma (lymphoma in African Children), diffuse large B-cell lymphoma and post-transplant lymphoma.

39. A method for improving B cell antigen presentation, wherein comprising the following steps: (1) culturing and expanding a B cell, the B cell carries an EBV antigen; (2) irradiating with the B cell obtained in the step (1) with a certain dose of ionizing irradiation.

40. The method according to claim 39, wherein the B cell is subject to immortalization treatment before the step (1).

41. The method according to claim 39, wherein the B cell retains biological activity but is incapable of proliferation after being irradiated with the ionizing irradiation.

42. A system for improving B cell antigen presentation, wherein comprising a cell culture and expansion device for expanding B cell carrying an EBV antigen; and an irradiation device for irradiating the B cell with ionizing irradiation lower than inactivation threshold.

43. The system for improving B cell antigen presentation according to claim 42, further comprising: a cryopreservation device for cryopreserving the B cell irradiated with the ionizing irradiation.
